# EUROPEAN PATENT APPLICATION

(11) **EP 2 425 846 A1**
(43) Date of publication of application: **07.03.2012**
(21) Application number: 11181114.7
(22) Date of filing: 13.07.2005
(51) Int. Cl.: A61K 38/00, A61K 31/445, A61K 31/55, A61K 38/09

(54) **Acetylcholinesterase Inhibitors and leuprolide acetate for the treatment of Alzheimer's disease**

(30) Priority: 23.12.2004 US 638123 P
(62) Divisional of application: 05771273.9
(71) Applicant: Voyager Pharmaceutical Corporation, Alpharetta, GA 30004 (US)
(72) Inventor: Gregory, Christopher W., Cary, NC 27519 (US); Smith, Patrick S., Raleigh, NC 27614 (US)
(74) Representative: Harris, Jennifer Lucy

(57) **Abstract**

Methods of treating, mitigating, slowing the progression of, or preventing Alzheimer's Disease include administration of gonadotropin-releasing hormone analogues in combination with acetylcholinesterase inhibitors and/or N-methyl-D-aspartate receptor antagonists.

## Description

### RELATED APPLICATIONS

This application claims priority under 35 U.S.C. § 119 to U.S. Provisional Patent Application No. 60/638,123, filed December 23, 2004, the entirety of which is incorporated herein by reference.

### FIELD OF INVENTION

This invention relates to the treatment, mitigation, slowing the progression of, and prevention of Alzheimer's Disease.

### BACKGROUND

Alzheimer's disease (AD) is a neurodegenerative disorder that leads to progressive memory loss, impairments in behavior, language, and visuo-spatial skills, and ultimately death. The disease is invariably associated with and defined by neuronal and synaptic loss, the presence of extracellular deposits of β-amyloid protein, and intracellular formation of neurofibrillary tangles in the brain (Selkoe DJ. Alzheimer disease: Genotypes, phenotypes and treatments. Science 275:630-631, 1997; Smith MA. Alzheimer disease. In: Bradley RJ and Harris RA, eds. International Review of Neurobiology., Vol. 42. San Diego, CA: Academic Press, Inc.1-54, 1998). The etiology of AD is not known, although a number of hypotheses exists regarding the mechanisms of damage to the brain. There is a continuing need for cost-effective approaches for treating, mitigating, slowing the prevention of, and preventing AD.

### SUMMARY

Gonadotropin-releasing hormone (GnRH) analogues decrease blood and tissue levels of the gonadotropins follicle-stimulating hormone (FSH) and luteinizing hormone (LH). Acetylcholinesterase (AChE) inhibitors increase acetylcholine levels at neuronal synapses, and N-methyl-D-aspartate (NMDA) receptor antagonists decrease glutamate-stimulated excitotoxicity. According to the present invention, GnRH analogues in combination with AChE inhibitors and/or NMDA receptor antagonists are effective in treating, mitigating, slowing the progression of, and/or preventing AD.

In accordance with embodiments of the present invention, decreased blood and tissue levels, production, function, and activity of FSH and LH, along with AChE inhibition at neuronal synapses, prevent aborted cell cycling of terminally differentiated neurons and elevate the levels of acetylcholine in neuronal synapses of the basal forebrain, amygdala, hippocampus, and entorhinal cortex, thus treating, mitigating, slowing the progression of, and/or preventing AD.

In other embodiments of the invention, decreased blood and tissue levels, production, function, and activity of FSH and LH, along with decreased glutamate-stimulated excitotoxicity, prevent aborted cell cycling of terminally differentiated neurons and prevent neuronal death due to glutamate-induced neuronal excitotoxicity.

In other embodiments of the invention, decreased blood and tissue levels, production, function, and activity of FSH and LH, along with AChE inhibition at neuronal synapses and decreased glutamate-stimulated neuronal excitotoxicity, prevent aborted cell cycling of terminally differentiated neurons, elevate the levels of acetylcholine in neuronal synapses of the basal forebrain, amygdala, hippocampus, and entorhinal cortex, and prevent neuronal death due to glutamate-induced neuronal excitotoxicity.

An embodiment of the present invention provides a method of treating, mitigating, slowing the progression of, or preventing Alzheimer's Disease, comprising administering a therapeutically effective combination, or a therapeutically effective synergistic combination, of a gonadotropin-releasing hormone analogue (for example leuprolide acetate), and either or both of an acetylcholinesterase inhibitor (for example donepezil, rivastigimine, galantamine, or tacrine) and an N-methyl-D aspartate receptor antagonist (for example, memantine).

### BRIEF DESCRIPTION OF THE DRAWINGS

FIG. 1 presents results of a clinical trial comparing administration of a combination of an acetylcholinesterase inhibitor (ACI) and leuprolide acetate with administration of a combination of an ACI with placebo, using the Alzheimer's Disease Assessment Scale - Cognitive (ADAS-Cog) test.
FIG. 2 presents results of the same clinical trial, using the Alzheimer's Disease Cooperative Study - Activities of Daily Living (ADCS-ADL) test.
FIG. 3 presents results of the same clinical trial, using the Alzheimer's Disease Cooperative Study - Clinical Global Impression of Change (ADCS-CGIC) test.

### DETAILED DESCRIPTION

### The Gonadotropin Hypothesis of Alzheimer's Disease

The cell cycle hypothesis of AD, which is consistent with known abnormalities associated with the disease, proposes that AD is a result of aberrant re-entry of neurons into the cell cycle. Aberrant cell cycle re-entry has been proposed to be caused by an age-related upregulation of an unknown mitogen. The gonadotropin hypothesis proposes that LH is this mitogen.

LH and human chorionic gonadotropin (HCG) have been shown to be mitogenic in certain reproductive tissues (Horiuchi A, Nikaido T, Yoshizawa T, Itoh K, Kobayashi Y, Toki T, et al. HCG promotes proliferation of uterine leiomyomal cells more strongly than that of myometrial smooth muscle cells in vitro. Molec. Human Reprod. 6:523-528, 2000; Davies BR, Finnigan DS, Smith SK, and Ponder BA. Administration of gonadotropins stimulates proliferation of normal mouse ovarian surface epithelium. Gynecol. Endocrinol. 13:75-81, 1999; Webber RJ and Sokoloff L. In vitro culture of rabbit growth plate chondrocytes. 1. Age-dependence of response to fibroblast growth factor and "chondrocyte growth factor." Growth. 45:252-268, 1981).

Further, HCG and LH are frequently expressed by tumor cells (Yokotani T, Koizumi T, Taniguchi R, Nakagawa T, Isobe T, Yoshimura M, et al. Expression of alpha and beta genes of human chorionic gonadotropin in lung cancer. Int. J. Cancer. 71:539-544, 1997; Krichevsky A, Campbell-Acevedo EA, Tong JY, and Acevedo HF. Immunological detection of membrane-associated human luteinizing hormone correlates with gene expression in cultured human cancer and fetal cells. Endocrinol. 136:1034-1039, 1995; Whitfield GK and Kourides IA. Expression of chorionic gonadotropin alpha- and beta-genes in normal and neoplastic human tissues: relationship to deoxyribonucleic acid structure. Endocrinol. 117:231-236, 1985).

In addition, LH has been shown to activate extracellular signal-regulated kinase (ERK) and mitogen-activated protein (MAP) kinase. (Srisuparp S, Strakova Z, Brudney A, Mukherjee S, Reierstad S, Hunzicker-Dunn M, et al. Signal transduction pathways activated by chorionic gonadotropin in the primate endometrial epithelial cells. Biol. Reprod. 68:457-464, 2003; Cameron MR, Foster JS, Bukovsky A, and Wimalasena J. Activation of mitogen-activated protein kinases by gonadotropins and cyclic adenosine 5'-monophosphates in porcine granulosa cells. Biol. Reprod. 55:111-119, 1996). Increased serum concentrations of LH also correlate to periods of rapid growth: fetal life, the subsequent first year of life, and puberty. Once reproductive maturity is reached, it is believed that the mitogenicity of LH is countered by newly produced sex steroids and inhibins. However, it is also believed that protection against the mitogenic effects of LH is lost with the age-related decline in reproductive function that results in a decrease in sex steroids and inhibins and an increase in LH. While this hormonal profile may be advantageous in the developing brain of a fetus, terminally differentiated adult neurons are likely to be unable to respond appropriately to mitogenic stimulus, resulting in the neuronal dysfunction and death characteristic of AD.

It has been shown *in vitro* and *in vivo* that gonadotropins modulate amyloid-β precursor protein processing and β-amyloid protein generation. (Bowen RL, Verdile G, Liu T, Parlow AF, Perry G, Smith MA, et al. Luteinizing hormone, a reproductive regulator that modulates the processing of amyloid-b precursor protein and amyloid-b deposition. J. Biol. Chem. 279:20539-20545, 2004). In addition, human granulosa cells stimulated with gonadotropins are characterized by upregulation of expression of the presenilin-1 and -2 genes, which code for proteins involved in amyloid-β precursor protein processing. (Rimon E, Sasson R, Dantes A, Land-Bracha A, and Amsterdam A. (2004) Gonadotropin-induced gene regulation in human granulosa cells obtained from IVF patients: modulation of genes coding for growth factors and their receptors and genes involved in cancer and other diseases. Int. J. Oncol. 24:1325-1338, 2004).

### Therapeutic Strategies Based on the Gonadotropin Hypothesis of AD

According to the present invention, drugs that inhibit gonadotropin synthesis and secretion should result in halting or slowing of the disease process of AD, and may lead to its mitigation or reversal. A therapeutic strategy for treating AD based on the gonadotropin hypothesis is disclosed in U.S. Patent No. 6,242,421, issued on June 5, 2001 to Richard L. Bowen, incorporated herein by reference.

There are a number of drugs approved by the United States Food and Drug Administration (FDA) that effectively suppress gonadotropins. These drugs fall into two classes: GnRH agonists (e.g., Zoladex^{®} brand of goserelin acetate) and GnRH antagonists (e.g., Plenaxis™ brand of abarelix). GnRH agonists were developed as a method of suppressing sex steroid production as an alternative to surgical castration in the treatment of advanced prostate cancer. GnRH agonists have since been used in a number of other hormone-related conditions, including endometriosis, uterine fibroids, and infertility, and are even approved for use in children suffering from precocious puberty (Filicori M, Hall DA, Loughlin JS, Vale W, and Crowley Jr. WF. A conservative approach to the management of uterine leiomyoma: pituitary desensitization by a luteinizing hormone-releasing hormone analogue. Amer. J. Obstetr. Gynecol. 147:726-727, 1983; Laron Z, Kauli R, Zeev ZB, Comaru-Schally AM, and Schally AV. D-TRP5-analogue of luteinising hormone releasing hormone in combination with cyproterone acetate to treat precocious puberty. Lancet. 2:955-956, 1981; Meldrum DR, Chang RJ, Lu J, Vale W, Rivier J, and Judd HL. "Medical oophorectomy" using a long-acting GNRH agonist-a possible new approach to the treatment of endometriosis. J. Clin. Endocrinol. Metabol. 54:1081-1083, 1982; Wildt L, Diedrich K, van der Ven H, al Hasani S, Hubner H, and Klasen R. Ovarian hyperstimulation for in-vitro fertilization controlled by GnRH agonist administered in combination with human menopausal gonadotropins. Human Reprod. 1:15-19, 1986).

For chronic use, GnRH agonists are usually more effective than GnRH antagonists at suppressing gonadotropins. GnRH antagonists were developed to inhibit gonadotropin and sex steroid synthesis and secretion without causing the initial spike or burst in gonadotropins and sex steroids typically associated with GnRH agonists. However, while GnRH antagonists may prevent this initial burst, there is usually more "breakthrough" in LH and testosterone secretion with use of GnRH antagonists than occurs with use of GnRH agonists. (Praecis Pharmaceuticals Incorporated, Plenaxis Package Insert. 2004.) This may be due to a compensatory increase in hypothalamic GnRH secretion, which alters the ratio of the competing ligands, resulting in activation of the GnRH receptor. In contrast, with GnRH agonists, a compensatory increase in hypothalamic GnRH would only serve to potentiate receptor down-regulation. In addition, GnRH antagonists are associated with occasional anaphylactic reactions due to their high histamine releasing properties. (Millar RP, Lu ZL, Pawson AJ, Flanagan CA, Morgan K, and Maudsley SR. Gonadotropin-releasing hormone receptors. Endocr. Rev. 25:235-275, 2004).

GnRH agonists are analogues of the endogenous GnRH decapeptide with specific amino acid substitutions. Replacement of the GnRH carboxyl-terminal glycinamide residue with an ethylamide group increases the affinity these analogues possess for the GnRH receptor as compared to the endogenous peptide. Many of these analogues also have a longer half-life than endogenous GnRH. Administration of GnRH agonists results in an initial increase in serum gonadotropin concentrations that typically persists for several days (there is also a corresponding increase in testosterone in men and estrogen in pre-menopausal women). The initial increase is typically followed by a precipitous decrease in gonadotropins. This suppression is secondary to the loss of GnRH signaling due to down-regulation of pituitary GnRH receptors (Belchetz PE, Plant TM, Nakai Y, Keogh EJ, and Knobil E. Hypophysial responses to continuous and intermittent delivery of hypothalamic gonadotropin-releasing hormone. Science. 202:631-633, 1978). This is believed to be a consequence of the increased concentration of ligand, the increased affinity of the ligand for the receptor, and the continuous receptor exposure to ligand as opposed to the intermittent exposure that occurs with physiological pulsatile secretion.

Since GnRH agonists are small peptides, they are generally not amenable to oral administration. Therefore, they are customarily administered subcutaneously, intra-muscularly, or via nasal spray. GnRH agonists are potent, with serum concentrations of less than 1 ng/ml of the GnRH agonist leuprolide acetate being considered to be adequate for testosterone suppression. (Fowler JE, Flanagan M, Gleason DM, Klimberg IW, Gottesman JE, and Sharifi R. Evaluation of an implant that delivers leuprolide for 1 year for the palliative treatment of prostate cancer. Urol. 55:639-642, 2000). Due to their small size and high potency, these peptides are strong candidates for use in long-acting depot delivery systems. At least five such products, each having a duration of action ranging from 1 month to 1 year, are currently marketed in the United States. Four of these products contain leuprolide acetate, and the fifth contains goserelin.

Leuprolide acetate has been on the market for close to two decades and continues to demonstrate a favorable side effect profile. Most of the side effects such as hot flashes and osteoporosis can be attributed to loss of sex steroid production (Stege R. Potential side-effects of endocrine treatment of long duration in prostate cancer. Prostate Suppl. 10:38-42, 2000). For treatment of female AD patients, sex steroid suppression should not be a major issue since such patients are post-menopausal and their estrogen production is already significantly decreased. However, since males in the same age group normally produce appreciable amounts of testosterone, add-back testosterone supplementation should counter symptoms associated with the suppression of testosterone.

The safety of GnRH agonists is further supported by the fact that an estimated well over 100 million doses have been administered to date (based on sales figures) with no serious consistent adverse effects. In addition, the low toxicity of GnRH agonists was demonstrated in a clinical trial in which men with prostate cancer received daily injections, for up to two years, that were twenty-fold higher (i.e., 20 mg per day) than the currently approved dose of 1 mg per day. The 20 mg dose did not result in any adverse effects different from what was seen with the 1 mg dose (TAP Pharmaceuticals, Inc., Lupron Depot 7.5 mg Package Insert. 2003). The safety profile of GnRH agonists along with delivery systems that promote compliance for long periods make these compounds well suited for the AD population.

### The Cholinergic Hypothesis of Alzheimer's Disease

The cholinergic hypothesis of AD proposes that cholinergic neurons in the basal forebrain degenerate, leading to decreased cholinergic neurotransmission in the cerebral cortex. These changes are thought to contribute to the learning and memory deficits associated with AD.

The enzyme acetylcholinesterase (AChE) hydrolyzes acetylcholine, thereby making it a suitable substrate for binding to the acetylcholine muscarinic and nicotinic receptors, which activate downstream signaling pathways in the cortical pyramidal neurons. In brains with AD, there is an alteration in neurotransmission resulting from reduced levels of acetylcholine. AChE breaks down the acetylcholine that is produced, thereby decreasing activation of postsynaptic acetylcholine muscarinic and nicotinic receptors, which is believed to result in decreased processing of amyloid precursor protein, increased amyloid-β production, and accumulation of hyperphosphorylated tau protein, all hallmarks of AD pathology. Inhibition of AChE enzyme activity is believed to reduce the breakdown of endogenously released acetylcholine, which is expected to result in increased activation of postsynaptic receptors with the end result of reversing the deleterious consequences described above.

### Therapeutic Strategies Based on the Cholinergic Hypothesis

Four AChE inhibitors are currently marketed to improve central cholinergic neurotransmission and are used to treat AD due to their positive effects on memory and cognitive impairment (Racchi M, Mazzucchelli M, Porrello E, Lanni C, Govoni S. Acetylcholinesterase inhibitors: novel activities of old molecules. Pharmacol. Res. 50:441-451, 2004). Donepezil (marketed under the name Aricept^{®}) is a piperidine-based, reversible AChE inhibitor that is highly selective for AChE. Rivastigmine (marketed under the name Exelon^{®}) is a carbamylating, pseudo-irreversible AChE inhibitor that shows dose-dependent cognitive and behavioral benefits in mild-to-moderate AD patients. Galantamine (marketed under the name Reminyl^{®}), a tertiary alkaloid, is a reversible, competitive AChE inhibitor that has been shown to produce beneficial effects on cognition and the ability to perform activities of daily living. Tetrahydroaminoacridine (tacrine) (marketed under the name Cognex^{®}), was the first acetylcholinesterase inhibitor approved for use in Alzheimer's patients. These compounds are available for the symptomatic treatment of patients with mild-to-moderate AD and are considered to be effective for short-term intervention. While the primary efficacy of this family of compounds likely results from the prevention of acetylcholine breakdown, recent work suggests that these drugs may also interfere with the amyloid cascade by preventing accumulation of amyloid-β (Giacobini E. Cholinesterase inhibitors stabilize Alzheimer disease. Neurochem. Res. 25:1185-1190, 2000).

### The Neuronal Glutamate Hypothesis of AD

Neuronal excitotoxicity resulting from glutamate overstimulation of the N-methyl-D-aspartate (NMDA) receptor may play a role in AD pathophysiology. Activation of the NMDA receptor is critical for normal cognitive function (Shimizu E, Tang YP, Rampon C, Tsien JZ. (2000) NMDA receptor-dependent synaptic reinforcement as a crucial process for memory consolidation [published correction in Science 2001, 291:1902]. Science 290:1170-1174, 2000). Overstimulation of the receptor by glutamate causes increased intracellular calcium and is implicated in neuronal death.

### Therapeutic Strategy Based on the Neuronal Glutamate Hypothesis

Memantine (marketed under the name Namenda^{®}), a noncompetitive antagonist with moderate affinity for the NMDA receptor, blocks neuronal toxicity caused by glutamate. Memantine is approved for use in treating moderate to severe AD.

### Combination Therapy for AD

Each of leuprolide acetate, AChE inhibitors, and NMDA receptor antagonists, when used separately, has a distinct mechanism of action. Treatment of mild to moderate AD patients with leuprolide acetate typically prevents the aberrant re-entry of terminal neurons into the cell cycle, thereby preventing neuronal cell death characteristic of AD brains. AChE inhibitors typically improve cholinergic neurotransmission in viable neurons. NMDA receptor antagonists typically prevent glutamate-induced neuronal toxicity. Concomitant use of memantine typically does not inhibit the action of acetylcholinesterase inhibitors.

According to the present invention, combining leuprolide acetate with AChE inhibitors is expected to prevent neuronal cell death and improve neurotransmission in surviving cells, resulting in improved cognitive functioning. Using leuprolide acetate in combination with NMDA receptor antagonists is expected to have the net effect of reducing the number of neurons that die in AD brains. Combination therapy with leuprolide acetate, AChE inhibitors, and NMDA antagonists is expected to prevent neuronal death caused by aberrant cycling and glutamate toxicity and improve cholinergic neurotransmission.

In accordance with embodiments of the present invention, decreased blood and tissue levels, production, function, and activity of FSH and LH, along with AChE inhibition at neuronal synapses, prevents aborted cell cycling of terminally differentiated neurons and elevates the levels of acetylcholine in neuronal synapses of the basal forebrain, amygdala, hippocampus, and entorhinal cortex, thus treating, mitigating, slowing the progression of, and/or preventing AD.

In other embodiments of the invention, decreased blood and tissue levels, production, function, and activity of FSH and LH, along with decreased glutamate-stimulated excitotoxicity, prevents aborted cell cycling of terminally differentiated neurons and prevents neuronal death due to glutamate-induced neuronal excitotoxicity, thus treating, mitigating, slowing the progression of, and/or preventing AD.

In other embodiments of the invention, decreased blood and tissue levels, production, function, and activity of FSH and LH, along with AChE inhibition at neuronal synapses and decreased glutamate-stimulated neuronal excitotoxicity, prevents aborted cell cycling of terminally differentiated neurons, elevates the levels of acetylcholine in neuronal synapses of the basal forebrain, amygdala, hippocampus, and entorhinal cortex, and prevents neuronal death due to glutamate-induced neuronal excitotoxicity, thus treating, mitigating, slowing the progression of, and/or preventing AD.

### Clinical Trials

During 2004-2005, a 48-week, double-blind placebo-controlled dose ranging study was conducted in 108 women diagnosed with mild-to-moderate Alzheimer's Disease. The study inclusion criteria included a requirement that each patient either (a) is taking a cholinesterase inhibitor, began taking it at least 90 days prior to the trial and is likely to continue taking it at the same dosage level throughout the trial; or (b) has never taken a cholinesterase inhibitor or has stopped taking at least 90 days prior to the trial and is likely to remain off cholinesterase inhibitors throughout the trial. The patients in the subgroup taking cholinesterase inhibitors were in turn divided into two groups for analysis purposes: Group 1 patients were administered an injectable 22.5 mg formulation of leuprolide acetate in combination with a stable dose of acetylcholinesterase inhibitors (AChEI); Group 2 patients were administered a placebo injection (saline) in combination with a stable dose of AChEI. The administrations of leuprolide acetate and placebo occurred at weeks 0, 12, 24, 36, and 48 of the study. As used in the study, a stable dose of AChEI meant that the patient took substantially the same formulation of AChEl, at substantially the same dosage amount and frequency, throughout the study period. At the completion of the study, Group 1 included 24 subjects and Group 2 included 26 subjects. The trial utilized the ADAS-Cog, an assessment of cognitive decline; the ADCS-ADL, an assessment of ability to perform activities of daily living; and the ADCS-CGIC, a clinician's assessment of the patient's cognitive state. These tests are commonly used assessments for primary endpoints in AD clinical trials.

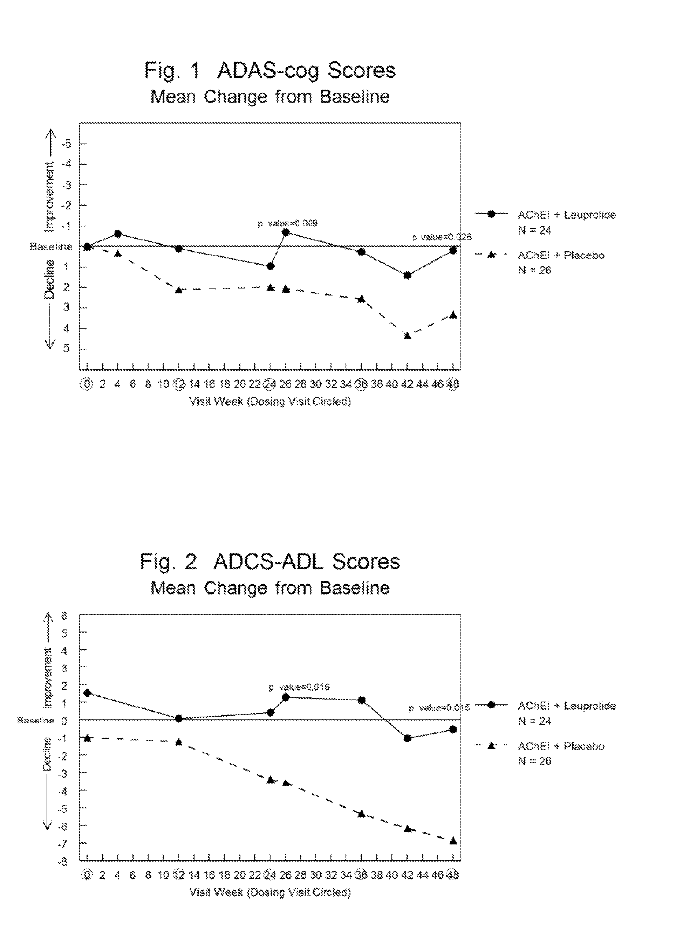

Table 1 below shows the mean scores of the study participants on the ADAS-Cog test, which are also depicted in FIG. 1, along with the applicable statistical p-levels:

**Table 1: ADAS-Cog Scores**

| | | Mean Change from Baseline | | | | | | |
|---|---|---|---|---|---|---|---|---|
| | Baseline | Wk. 4 | Wk. 12 | Wk. 24 | Wk. 26 | Wk. 36 | Wk. 42 | Wk. 48 |
| Group 1 | 20.31 | -0.62 | 0.10 | 0.95 | -0.69 | 0.26 | 1.41 | 0.18 |
| Group 2 | 24.29 | 0.31 | 2.09 | 1.98 | 2.03 | 2.53 | 4.32 | 3.30 |

Table 2 below shows the mean scores of the study participants on the ADCS-ADL test, which are also depicted in FIG. 2, along with the applicable p-levels:

**Table 2: ADCS-ADL Scores**

| | Mean Change from Baseline | | | | | | |
|---|---|---|---|---|---|---|---|
| | Wk. 4 | Wk. 12 | Wk. 24 | Wk. 26 | Wk. 36 | Wk. 42 | Wk. 48 |
| Group 1 | 1.54 | 0.08 | 0.42 | 1.29 | 1.13 | -1.04 | -0.54 |
| Group 2 | -1.00 | -1.23 | -3.38 | -3.54 | -5.31 | -6.15 | -6.85 |

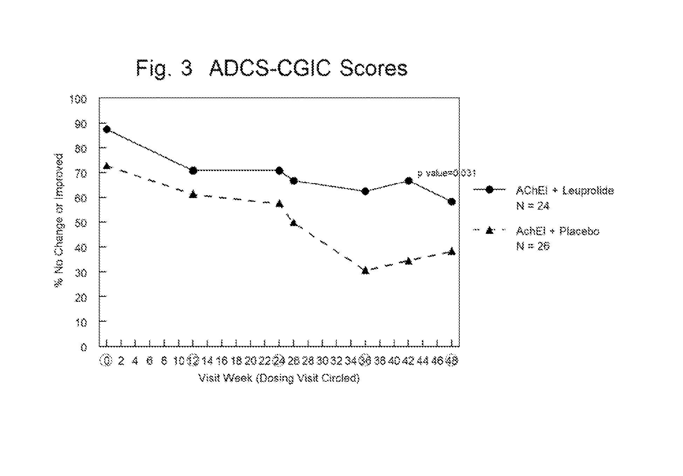

Table 3 reflects the scores of the study participants on the ADCS-CGIC test, which are also shown in FIG. 3, along with the applicable p-levels. Specifically, Table 3 and FIG. 3 show the proportion (percent) of patients in each group showing no change or improvement on the ADCS-CGIC test at various observation times during the trial.

**Table 3: ADCS CGIC Scores**

| | Percent of Subjects Scoring No Change or Improvement | | | | | | |
|---|---|---|---|---|---|---|---|
| | Wk. 4 | Wk. 12 | Wk. 24 | Wk. 26 | Wk. 36 | Wk. 42 | Wk. 48 |
| Group 1 | 87.5 | 70.8 | 70.8 | 66.7 | 62.5 | 66.7 | 58.3 |
| Group 2 | 73.0 | 61.5 | 57.7 | 50.0 | 30.8 | 34.6 | 38.5 |

An analysis of these data indicates, at statistically significant levels, that the mean ADAS-Cog scores for Group 1 (combination of AChEI and 22.5 mg dosage of leuprolide acetate) remained essentially baseline (a decline of 0.18 points) compared to a decline of 3.3 points in the placebo group (Group 2), with an unadjusted p-value of 0.026. The mean ADCS-ADL score in Group 1 also remained essentially at baseline (a decline of 0.54 points) compared to a decline in the placebo group (Group 2) of 6.85 points, with an unadjusted p-value of 0.015. In the ADCS-CGIC tests, 58% of the patients in Group 1 scored "no change" or "improvement" at week 48, versus 38% of the patients in Group 2.

Table 4 shows the results on the ADAS-cog (mean change from baseline), ADCS-ADL (mean change from baseline) and ADAS-CGIC tests (percent no change or improvement) for a group of patients (N=12) administered an injectable 22.5 mg formulation of leuprolide acetate at 12-week intervals over a 48-week period.

**Table 4: Leuprolide Acetate without AChEI Inhibitor**

| | Baseline | Wk. 4 | Wk. 12 | Wk. 24 | Wk. 26 | Wk. 36 | Wk. 42 | Wk. 48 |
|---|---|---|---|---|---|---|---|---|
| ADAS-cog | 19.79 | 2.17 | 2.99 | 3.94 | 1.20 | 3.24 | 5.22 | 4.68 |
| ADCS-ADL | | -2.75 | -1.92 | -4.83 | -4.58 | -5.17 | -5.17 | -6.50 |
| ADCS-CGIC | | 66.7% | 50% | 41.7% | 41.7% | 50% | 50% | 25% |

Analysis of these data also suggests that the combination of leuprolide acetate with acetylcholinesterase inhibitors has a greater effect on preventing or slowing the progress of AD than the additive effects of the two drugs administered alone.

The clinical trial also involved AD patients who were using NMDA receptor antagonists concomitantly with leuprolide acetate. Anecdotal evidence from the trial also suggests that the use of a combination of leuprolide acetate and NMDA receptor antagonists also has a greater effect on preventing or slowing the progress of AD than the additive effects of the two drugs administered separately.

### Formulations

As mentioned above, GnRH agonists are small peptides, and as such are generally not amenable to oral administration. Therefore, they are customarily administered subcutaneously, intra-muscularly, or via nasal spray. In an embodiment, the leuprolide acetate is provided for administration in a formulation, obtained from Durect Corporation of Cupertino, California under the trade name DURIN. This formulation is a solid formulation comprising approximately 25-30 weight % leuprolide acetate dispensed in a matrix of poly (DL-lactide-co-glycolide). The formulation is a cylindrical, opaque rod with nominal dimensions of approximately 1.5mm (diameter) by approximately 2.0cm (length). This formulation is designed to be implanted into the patent about every two months, to provide approximately 11.25mg leuprolide per 2 cm rod, and to provide a substantially uniform release profile. Leuprolide acetate is metabolized by peptidases, and the cytochrome P450 enzymes are not involved.

Acetylcholinesterase inhibitors and NMDA receptor antagonists are orally available and generally delivered in tablet or liquid form. Donepezil is metabolized by cytochrome P450 enzymes into multiple metabolites. Rivastigmine is metabolized through the action of hydrolysis by esterases. Galantamine is metabolized by hepatic cytochrome P450 enzymes. Tacrine is metabolized by cytochrome P450 enzymes into multiple metabolites. Memantine undergoes little metabolism, with the majority (up to 82%) of a dose being excreted in the urine unchanged; the remainder is converted to three polar metabolites.

Given the different availabilities and routes of metabolism, it is expected that two or more of GnRH agonists, AChE inhibitors, and NMDA receptor antagonists will be administered in a combination therapy that may or may not be in a single dosage form.

Aspects and features of the present disclosure are set out in the following numbered clauses which contain the subject matter of the claims of the parent application as originally filed.
1. A method of treating, mitigating, slowing the progression of, or preventing Alzheimer's disease, comprising the step of:
   administering a therapeutically effective combination of a gonadotropin-releasing hormone analogue with an acetylcholinesterase inhibitor or an N-methyl-D-aspartate receptor antagonist.
2. A method of reducing occurrence of aborted cell cycling of terminally differentiated neurons of a patient, comprising the step of:
   administering a therapeutically effective combination of a gonadotropin-releasing hormone analogue with at least one of an acetylcholinesterase inhibitor and an N-methyl-D-aspartate receptor antagonist.
3. A method of treating, mitigating, slowing the progression of, or preventing Alzheimer's disease, comprising the step of:
   administering a therapeutically effective amount of leuprolide acetate in combination with at least one of a therapeutically effective amount of an acetylcholinesterase inhibitor and a therapeutically effective amount of an N-methyl-D-aspartate receptor antagonist.
4. A method of treating, mitigating, slowing the progression of, or preventing Alzheimer's disease, comprising the step of:
   administering a therapeutically effective synergistic combination of a gonadotropin-releasing hormone analogue with an acetylcholinesterase inhibitor or an N-methyl-D-aspartate receptor antagonist.
5. The method of clause 1, wherein the gonadotropin-releasing hormone is leuprolide acetate, and the acetylcholinesterase inhibitor is a selected from the group consisting of donepezil, rivastigimine, galantamine and tacrine.
6. The method of clause 2, wherein the gonadotropin-releasing hormone is leuprolide acetate, and the acetylcholinesterase inhibitor is a selected from the group consisting of donepezil, rivastigimine, galantamine and tacrine.
7. The method of clause 1, wherein the gonadotropin-releasing hormone is leuprolide acetate, and the N-methyl-D-aspartate receptor antagonist is memantine.
8. The method of clause 2, wherein the gonadotropin-releasing hormone is leuprolide acetate, and the N-methyl-D-aspartate receptor antagonist is memantine.
9. The method of clause 3, wherein the therapeutically effective amount of leuprolide acetate is administered in combination with a therapeutically effective amount of an acetylcholinestarase inhibitor selected from the group consisting of donepezil, rivastigimine, galantamine and tacrine and a therapeutically effective amount of an N-methyl-D-aspartate receptor antagonist.
10. The method of clause 9, wherein the N-methyl-D-aspartate receptor antagonist is memantine.
11. The method of clause 4, wherein the therapeutically effective synergistic combination is a therapeutically effective synergistic combination of leuprolide acetate and an acetylcholinestarase inhibitor selected from the group consisting of donepezil, rivastigimine, galantamine and tacrine.
12. The method of clause 4, wherein the therapeutically effective synergistic combination is a therapeutically effective synergistic combination of leuprolide acetate and memantine.
13. The method of any of clauses 1-12, wherein the gonadotropin-releasing hormone analogue comprises leuprolide and is administered approximately once every 60 days in combination with a stable dose of an acetylcholinesterase inhibitor.
14. The method of any of clauses 1-12, wherein the combination comprises approximately 22.5 mg of leuprolide acetate.
15. The method of clause 14, wherein the leuprolide acetate is administered in a controlled-release formulation.
16. A combination comprising:
   a gonadotropin-releasing hormone analogue and at least one of an acetylcholinesterase inhibitor and an N-methyl-D-aspartate receptor antagonist.
17. The combination of clause 16, wherein the gonadotropin-releasing hormone comprises leuprolide acetate and the acetylcholinesterase inhibitor is selected from the group consisting of donepezil, rivastigimine, galantamine and tacrine.
18. The combination of clause 16, wherein the gonadotropin-releasing hormone comprises leuprolide acetate and the N-methyl-D-aspartate receptor antagonist comprises memantine.

While various embodiments of the present invention have been described above, it should be understood that they have been presented by way of example only, and not by way of limitation. The breadth and scope of the present invention should not be limited to any of the above-described exemplary embodiments, but should be defined in accordance with the appended claims.

## Claims

1. A combination of a gonadotropin-releasing hormone agonist and an acetylcholinesterase inhibitor for use in a method of treating, mitigating, slowing the progression of, or preventing Alzheimer's disease or reducing occurrence of aborted cell cycling of terminally differentiated neurons of a patient.

2. The use of a combination of a gonadotropin-releasing hormone agonist and an acetylcholinesterase inhibitor in the manufacture of a medicament for treating, mitigating, slowing the progression of, or preventing Alzheimer's disease or reducing occurrence of aborted cell cycling of terminally differentiated neurons of a patient.

3. The combination of claim 1 or use of claim 2, wherein the acetylcholinesterase inhibitor is selected from the group consisting of galantamine, rivastigimine, tacrine and donepezil.

4. The combination of claim 1 or 3, or use of claim 2 or 3, wherein the gonadotropin-releasing hormone agonist is leuprolide acetate.

5. The combination or use of claim 4, wherein the leuprolide acetate is provided for administration approximately once every 60 days in combination with a stable dose of an acetylcholinesterase inhibitor.

6. The combination or use of claim 4 or 5, wherein the combination comprises approximately 22.5 mg of leuprolide acetate.

7. The combination or use of claim 6, wherein the leuprolide acetate is provided for administration in a controlled-release formulation.

8. The combination of any one of claims 1 and 3-7, or use of any one of claims 2-7, wherein the gonadotropin-releasing hormone agonist is provided for administration subcutaneously, intra-muscularly, or via nasal spray.

9. The combination of any one of claims 1 and 3-8, or use of any one of claims 2-8, wherein the acetylcholinesterase inhibitor is provided for administration orally.

10. The combination of anyone of claims 1 and 3-9, or use of a combination of any one of claims 2-9, wherein the combination additionally comprises a N-methyl-D-aspartate receptor antagonist.

11. The combination or use of claim 10, wherein the N-methyl-D-aspartate receptor antagonist is memantine.
